# EUROPÄISCHE PATENTANMELDUNG

(11) **EP 3 593 753 A1**
(43) Veröffentlichungstag der Anmeldung: **15.01.2020**
(21) Anmeldenummer: 18182507.6
(22) Anmeldetag: 09.07.2018
(51) Int. Cl.: A61C 13/00, A61K 6/02, A61C 13/08, A61C 13/083

(54) **MEHRSCHICHTIGER FORMKÖRPER**

(71) Anmelder: VITA-ZAHNFABRIK H. Rauter GmbH & Co. KG, 79713 Bad Säckingen (DE)
(72) Erfinder: Bojemüller, Enno, 79713 Bad Säckingen (DE); Gorbar, Michael, 8600 Dübendorf (CH); Penner, Dirk, 8048 Zürich (CH); Tholey, Michael, 79713 Bad Säckingen (DE)
(74) Vertreter: dompatent von Kreisler Selting Werner - Partnerschaft von Patent- und Rechtsanwälten mbB

(57) **Zusammenfassung**

Es wird ein Formkörper mit einer Vielzahl aufeinander angeordneter keramischer Lagen sowie ein Verfahren zu dessen Herstellung beschrieben. Des Weiteren wird die Verwendung eines mehrschichtigen Formkörpers zur Herstellung einer dentalen Restauration beschrieben.

## Beschreibung

Die vorliegende Erfindung betrifft einen Formkörper mit einer Vielzahl aufeinander angeordneter keramischer Lagen sowie ein Verfahren zu dessen Herstellung. Darüber hinaus betrifft die Erfindung die Verwendung eines mehrschichtigen Formkörpers zur Herstellung einer dentalen Restauration.

Keramische Materialien zeichnen sich durch eine Vielzahl von Eigenschaften aus, die mit anderen Materialien nicht zu realisieren sind, wie beispielsweise Härte, Langlebigkeit, Abriebfestigkeit, Korrosionsbeständigkeit und thermische und elektrische Isolation.

Aufgrund ihrer unterschiedlichen Eigenschaften finden keramische Materialien eine breite Anwendung, von der Herstellung von Alltagsgegenständen wie Geschirr oder Fliesen bis hin zu einer Verwendung in der Elektronik, Informationstechnologie oder als biomedizinische Prothesen. Besonders durch die fortschreitende Entwicklung der CAD/CAM-Technologie sind direkt bearbeitbare oder nach der Bearbeitung dichtsinterbare Keramiken zunehmend in den Fokus gerückt und drängen in den Bereich der händisch hergestellten Zahnrestaurationen.

Im Gegensatz zu Metallen weisen Keramiken meist spröde Materialeigenschaften auf, die durch geeignete Stabilisierungsmechanismen oder Materialzusammensetzungen optimiert werden können. Als Beispiele können hierzu mit Yttriumoxid stabilisiertes Zirkoniumdioxid oder polymerinfiltrierte Keramiken (VITA ENAMIC®) dienen. Besondere Beachtung bei der Herstellung von keramischen Dentalrestaurationsmaterialien findet der zur Erlangung der erforderlichen Stabilität durchgeführte Sinterprozess. Die hier auftretende Sinterschrumpfung muss exakt beherrscht werden, um die gewünschten Materialeigenschaften reproduzierbar einstellen zu können. Um eine entsprechende Formgebung zu gewährleisten, werden Keramiken meist mit Presshilfsmitteln zu rieselfähigen Pressgranulaten verarbeitet, die auf automatischen Pressen zu Formkörpern wie Blöcke oder Discs verpresst werden. Bei größeren Formkörpern kann noch eine isostatische Nachverdichtung erfolgen, um Gefügeinhomogenitäten zu minimieren. Im Falle des Yttrium-stabilisierten Zirkoniumdioxids erfolgt eine Entbinderung und eine anschließende Sinterung auf eine poröse Stufe, in der das Material per CAD/CAM-Verfahren bearbeitet werden kann. Die dentale Restauration wird vergrößert ausgeschliffen und anschließend dicht gesintert, um die erforderliche Passung und auch die gewünschten Materialeigenschaften zu erlangen. Auch die Farbe der Restauration wird durch den Schritt des Dichtsinterns erreicht. Im Falle eines opaken Zirkoniumdioxids mit z.B. 3 mol-% tetragonaler Stabilisierung durch Yttriumoxid wird die so erhaltene Restauration anschließend mit einer Verblendkeramik händisch verblendet. Im Falle z.B. einer zunehmend kubischen 6 mol-% Stabilisierung mit Yttriumoxid kann das gesinterte Endprodukt aufgrund der höheren Transluzenz nach dem Dichtsintern schon die gewünschte Zahnfarbe aufweisen und braucht anschließend nicht noch verblendet zu werden.

Im Falle von polymerinfiltrierter Keramik (Hybridkeramik, Vita ENAMIC®) wird die keramische Komponente ebenfalls mit Presshilfsmitteln versehen, entbindert und auf die gewünschte Porosität gesintert. Im folgenden Schritt wird das durchgehende poröse Netzwerk mit einer mit einem Polymerisationsstarter versetzen Monomermischung infiltriert und polymerisiert, dass keine Hohlräume im Gefüge verbleiben. Dieses Hybridmaterial weist jetzt die endgültige Zahnfarbe auf und kann direkt per CAD/CAM-Verfahren zur dentalen Restauration verarbeitet werden.

Eine besondere Schwierigkeit bei der Herstellung dentaler Restaurationen liegt darin, dass natürliche Zähne nicht einfarbig sind, sondern einen komplexen Farbverlauf aufweisen, der berücksichtigt werden muss, um ein ästhetisch zufriedenstellendes Ergebnis zu erzielen. So können sich verschiedene Bereiche desselben Zahns in ihrer Farbe und ihrer Transparenz voneinander unterscheiden. Im Allgemeinen erstreckt sich der Farb- und Transparenzverlauf von transparent im okklusalen Bereich bis gelblich opak im zervikalen Bereich. Zur Erzielung einer gewünschten Farbgebung können Dentalrestaurationen nachträglich verblendet werden. Eine solche Verblendung beinhaltet jedoch üblicherweise einen hohen manuellen Aufwand und erfordert eine zahntechnische Ausbildung in den verwendeten Verblendmaterialien. Zusätzlich unterscheiden sich auch die mechanischen Eigenschaften im Zahn, das elastischere Dentin weist eine deutlich geringere Biegefestigkeit auf als der deutlich sprödere Zahnschmelz, mit dem die Zerkleinerung der Speisen erfolgt. Zusammen ergeben beide Komponenten aber einen hochbelastbaren Zahn, dessen flexiblerer Innenteil und die Wurzel, das Dentin, die in den Zahnschmelz aufgebrachten Kräfte abfedert. Auch diese Eigenschaften müssen von der dentalen Restauration widergespiegelt werden. Aufgrund dieser Ansprüche und vor dem Hintergrund einer zunehmenden Rationalisierung ist die Verwendung von Formkörpern zur Herstellung von dentalen Restaurationen bevorzugt, die auch ohne Verblendung ästhetischen und mechanischen Ansprüche gerecht werden und eine Farbgebung aufweisen, die in Farbe und Transparenz sowie Farbverlauf den natürlichen Zähnen möglichst nahe kommt.

EP 1 900 341 offenbart Formkörper aus mehreren unterschiedlich gefärbten Schichten mit spezieller Schichtabfolge, damit der Farbübergang zwischen den Schichten nicht sichtbar ist. Die Formkörper werden durch Trockenpressen von aufeinander geschichteten unterschiedlich gefärbten Glaskeramik-Pulvern, Entbinderung und Sinterung hergestellt. Aus diesem Formkörper können mittels CAD/CAM-Verfahren dentale Restaurationen hergestellt werden.

WO 2008/144388 stellt mehrschichtige keramische Körper und Systeme bereit, die mittels Bandguss- und Reaktionsverbindungs-Technologien hergestellt werden und eine verbesserte strukturelle Zuverlässigkeit im Vergleich zu keramischen Zusammensetzungen aufweisen, die mittels herkömmlicher Methoden hergestellt wurden. Die beschriebenen keramischen Körper und Systeme können für jede Art biomedizinischer Prothesen verwendet werden und sind besonders als dentale Restaurationen nützlich.

WO 2008/083358 beschreibt einen Dentalrohling, der mindestens eine innere Zone oder Schicht einer ersten Farbe und eine äußere Zone oder Schicht einer zweiten Farbe aufweist, wobei die innere und die äußere Zone konzentrisch angeordnet sind. Die innere Zone kann zur Gänze von der äußeren Zone umschlossen werden, so dass nur die äußere Zone auf allen Oberflächen des Rohlings sichtbar ist, nicht aber die innere Zone. Alternativ können sich die innere und die äußere Zone zu der gleichen Oberfläche des Rohlings hin erstrecken, so dass nur die äußere Zone die verbleibenden Oberflächen des Rohlings bedeckt. Der dentale Rohling kann außerdem eine Zwischenzone zwischen der inneren und der äußeren Zone aufweisen, wobei die Zwischenzone zur Gänze von der äußeren Zone umschlossen wird und/oder die Zwischenzone die innere Zone zur Gänze umschließt.

Die im Stand der Technik beschriebenen Formkörper weisen den Nachteil auf, dass zwischen zwei unterschiedlich eingefärbten Schichten ein teilweise sichtbarer Farbübergang auftritt und bei transluzenten Materialien weiterhin das Problem auftritt, dass zwischen den Schichten unterschiedlicher Einfärbung eine Graufärbung auftritt, die als dunkler oder heller Strich in der dentalen Restauration erkennbar ist. Darüber hinaus lassen sich mit herkömmlichen Pressverfahren nur eine begrenzte Anzahl von Schichten übereinander anordnen, da die Zahl der in der Software zur Verfügung stehenden Kanäle zum Bewegen einer zur Herstellung notwendigen Füllvorrichtung begrenzt ist. Auch das limitierte Platzangebot im Pressraum lässt zudem nicht beliebig viele unterschiedlich eingefärbte Pressgranulate zu, so dass neben der Begrenzung der Granulat- und Schichtdicken ein natürlicher Farbverlauf auf diese Weise nicht zugänglich ist.

Es besteht daher weiterhin ein Bedarf an keramischen Formkörpern, die modulierbare Eigenschaften aufweisen, insbesondere im Bereich der Dentalrestaurationen, um den natürlichen Farbverlauf und die natürlichen mechanischen Eigenschaften eines Zahns abzubilden, so dass sich die dentale Restauration in das vorhandene Zahngefüge ästhetischen Anforderungen entsprechend einfügt.

Es ist daher eine Aufgabe der vorliegenden Erfindung einen keramischen Formkörper zur Verfügung zu stellen, dessen Eigenschaften den individuellen optischen und funktionellen Bedürfnissen der jeweiligen Anwendung angepasst werden können.

Die Aufgabe wird durch einen Formkörper gelöst, der über eine Vielzahl aufeinander angeordneter keramischer Schichten verfügt. Es wurde überraschend gefunden, dass durch einen schichtartigen Aufbau sowohl die optischen Eigenschaften des Formkörpers, wie beispielsweise der Farbverlauf, als auch mechanische und strukturelle Eigenschaften, wie beispielsweise das Sinterverhalten, angepasst werden können.

Daher ist ein erster Gegenstand der vorliegenden Erfindung ein Formkörper mit einer Vielzahl aufeinander angeordneter keramischer Lagen, wobei mindestens 21 Lagen aufeinander angeordnet sind.

Unter keramischen Lagen im Sinne der vorliegenden Erfindung sind Lagen oder Schichten im Formkörper zu verstehen, die mindestens ein keramisches Material umfassen.

Um die Eigenschaften des Formkörpers möglichst präzise einstellen zu können, hat es sich als vorteilhaft erwiesen, wenn die Anzahl der keramischen Lagen des Formkörpers mehr als 21 beträgt, vorzugsweise mehr als 25, besonders bevorzugt mehr als 50 und insbesondere mehr als 100.

Vorteilhaft, insbesondere hinsichtlich der optischen Eigenschaften, ist weiterhin, wenn die Schichtdicke der einzelnen Lagen eine gewisse Stärke nicht überschreiten. Auf diese Weise kann eine kontinuierliche Änderung der Eigenschaften der verschiedenen Lagen, wie ein Farbverlauf erreicht werden, so dass die Übergänge zwischen den einzelnen Lagen mit dem bloßen Auge nicht mehr erkennbar sind. Daher weisen in einer bevorzugten Ausführungsform des erfindungsgemäßen Formkörpers die Lagen eine Stärke von 1 µm bis 120 µm, insbesondere 1 bis 99 µm, vorzugsweise 10 µm bis 80 µm, besonders bevorzugt30 µm bis 60 µm auf.

Die Eigenschaften des Formkörpers können dadurch eingestellt werden, dass die keramischen Lagen unterschiedliche Eigenschaften aufweisen, wie beispielsweise Einfärbung, Dichte, Porosität, Absorptionsverhalten und Röntgenopazität, oder dadurch, dass unterschiedliche Materialien verwendet werden. Daher ist eine Ausführungsform bevorzugt, in der die aufeinander angeordneten Lagen des erfindungsgemäßen Formkörpers jeweils dasselbe keramische Material umfassen oder mindestens eine der aufeinander angeordneten keramischen Lagen ein von den anderen keramischen Lagen verschiedenes keramisches Material umfasst.

Vorzugsweise umfassen die keramischen Lagen des erfindungsgemäßen Formkörpers ein keramisches Material ausgewählt aus der Gruppe bestehend aus Feldspatkeramik, Metalloxidkeramik, nicht-oxidische Keramik, polymerinfiltrierten Hybridkeramiken, Glaskeramik und Kombinationen davon. In einer besonders bevorzugten Ausführungsform ist das keramische Material ausgewählt aus der Gruppe bestehend aus Lithiumsilikatkeramik, Aluminiumoxidkeramik, Zirkonoxidkeramik, stabilisierte Zirkonoxidkeramik, Leuzitkeramik, Cordieritkeramik, durch Kristallite verstärkte Glaskeramiken, faserverstärkte Keramiken und Kombinationen davon. Diese Ausführungsformen können in dicht gesinterter Form oder auch in porös gesinterter Form, mit einem anschließenden Infiltrationsschritt, eingesetzt werden.

Die erfindungsgemäßen Formkörper erlauben die Herstellung von dentalen Restaurationen mit einem natürlichen Erscheinungsbild, die sich in die Umgebung natürlicher Zähne ohne optische Unterschiede einpassen. Insbesondere durch die Verwendung unterschiedlicher Materialien wurde überraschend gefunden, dass der farbliche Übergang in der Restauration stufenlos realisiert werden kann. Daher ist eine Ausführungsform besonders bevorzugt, in der der erfindungsgemäße Formkörper aus sich abwechselnden Schichten aus Zirkoniumdioxid und Feldspat aufgebaut ist.

Formkörper, die insbesondere zur Herstellung dentaler Restaurationen verwendet werden, müssen eine hohe mechanische Belastbarkeit aufweisen, die den Kräften, die während des Kauprozesses auf die Restauration wirken, standhält. Um eine entsprechende Belastbarkeit zu erreichen, hat es sich als vorteilhaft erwiesen, wenn der Formkörper dicht gesinterte Keramikpartikel umfasst. Daher ist eine Ausführungsform des erfindungsgemäßen Formkörpers bevorzugt, bei der mindestens eine der keramischen Lagen dicht gesinterte Keramikpartikel aufweist.

Weiterhin bevorzugt ist eine Ausführungsform des erfindungsgemäßen Formkörpers, in der die keramischen Lagen unterschiedliche Dichten aufweisen. Besonders bevorzugt ist ein Formkörper, in dem die keramischen Lagen unterschiedlicher Dichte einen Dichtegradienten bilden. Unter Dichtegradient im Sinne der vorliegenden Anmeldung ist eine kontinuierliche Veränderung der Dichte über den gesamten Formkörper oder über einen Teil des Formkörpers zu verstehen, wobei der Dichtegradient durch die Anordnung mehrerer Schichten unterschiedlicher Dichte aufeinander zustande kommt. Um die mechanischen und optischen Eigenschaften des erfindungsgemäßen Formkörpers anzupassen, kann es von Vorteil sein, wenn der Formkörper neben dem oder den keramischen Material(ien) weitere Materialen umfasst, die in das keramische Gerüst eingebaut werden. Dies ist insbesondere für Anwendungen in der Dentalrestauration von Vorteil, da auf diese Weise ein Gleichgewicht zwischen der erforderlichen mechanischen Belastbarkeit und einer guten Bearbeitbarkeit erreicht werden. Auch auf anderen Gebieten, wie beispielsweise der Elektronik, kann eine solche Kombinationsmöglichkeit verschiedener Materialien von Vorteil sein. Geeignete Methoden zur Herstellung solcher Materialien sind beispielsweise in den Schutzschriften WO 2002/076907, EP 1 238 956 und WO 2010/029515 beschrieben, auf die hier ausdrücklich Bezug genommen wird.

Entsprechend ist eine Ausführungsform des erfindungsgemäßen Formkörpers bevorzugt, in der mindestens eine der keramischen Lagen durch porös gesinterte Keramikpartikel gebildete Poren aufweist. Auf diese Weise kann nicht nur die Dichte des Formkörpers variiert werden. Die Poren ermöglichen auch den Einbau von weiteren Materialien, wie beispielsweise Polymeren, in das Keramikgerüst.

In einer bevorzugten Ausführungsform sind die Poren der keramischen Lagen umfassend porös gesinterte Keramikpartikel vollständig oder zumindest teilweise mit einem weiteren Material gefüllt. Besonders bevorzugt ist das weitere Material ausgewählt aus der Liste bestehend aus Kunststoff, Keramik, Glas und Metall. Für den Fall, dass es sich bei dem weiteren Material um eine Keramik handelt unterscheidet sich diese Keramik vorzugsweise von dem keramischen Material der Lagen des erfindungsgemäßen Formkörpers.

Vorzugsweise weisen die Poren eine Porengröße von 10 nm bis 100 µm auf, können aber in besonderen Ausführungsformen auch davon abweichen. Durch die Kombination von Schichten mit unterschiedlicher Porosität treten in den einzelnen Lagen unterschiedliche Porengrößen auf. Bei den Porengrößen handelt es sich um mittlere Porengrößen, die im Normalfall eine Größenverteilung aufweisen.

Weiterhin bevorzugt ist eine Ausführungsform des erfindungsgemäßen Formkörpers, in der eine Mehrzahl von keramischen Lagen mit porös gesinterten Keramikpartikeln jeweils in direkter Nachbarschaft aufeinander angeordnet sind unter Ausbildung eines sich durch die verschiedenen Lagen erstreckenden porösen Systems. Vorzugsweise weisen mindestens 70% der keramischen Lagen porös gesinterte Keramikpartikel auf, vorzugsweise mindestens 80% und bevorzugt 85% bis 99%.

In einer bevorzugten Ausführungsform weist der erfindungsgemäße Formkörper eine Porosität von 5 - 99 %, vorzugsweise 7 bis 95%, besonders bevorzugt 10 bis 90% auf. In einer ebenfalls bevorzugten Ausführungsform weist mindestens eine der keramischen Lagen eine Porosität von 5 - 99 %, vorzugsweise 7 bis 95%, besonders bevorzugt 10 bis 90% auf. In einer besonders bevorzugten Ausführungsform weisen mindestens zwei der keramischen Lagen unterschiedliche Porositäten auf.

Die physikalischen und chemischen Eigenschaften des erfindungsgemäßen Formkörpers können dadurch beeinflusst werden, dass dem keramischen Material Zuschlagstoffe beigefügt werden, wobei die Art und Menge der Zuschlagstoffe in den verschiedenen keramischen Lagen variieren kann. In einer bevorzugten Ausführungsform weist daher mindestens eine keramische Lage mindestens einen Zuschlagstoff zur Einstellung von physikalischen Eigenschaften wie Farbe, Transluzenz, Opazität, Röntgenopazität, Opaleffekten, Fluoroeszenz, Biegefestigkeit und/oder Elastizitätsmodul auf, wobei diese Aufzählung als beispielhaft zu verstehen ist.

In einer bevorzugten Ausführungsform weist der erfindungsgemäße Formkörper oder mindestens eine seiner Schichten weitere Zuschlagstoffe, insbesondere solche, die aus der Gruppe ausgewählt sind, die aus niedrig- oder hochschmelzenden Fritten oder Chemikalien wie Natriumcarbonat oder Borax und leuzithaltigen Zusätzen besteht, auf. Es wurde gefunden, dass durch Zugabe von geeigneten Zusätzen wie niedrig- oder hochschmelzenden Fritten oder Chemikalien wie z.B. Natriumcarbonat oder Flussmitteln wie z.B. Borax die Sintereigenschaften beeinflusst werden können. Leuzithaltige Zusätze können verwendet werden, um den Wärmeausdehnungskoeffizienten der Keramik zu beeinflussen.

Vorzugsweise ist der Zuschlagstoff ausgewählt aus der Gruppe bestehend ausfärbenden Substanzen, Keimbildnern, Flussmitteln, Läuterungsmitteln, keramischen Fasern, Nanomaterialien, Stabilisatoren und Mischungen hiervon. Neben dem Bedarf für die Formkörper selbst, besteht auch ein Bedarf für ein Verfahren, dass die Herstellung der beschriebenen Formkörper ermöglicht.

Daher ist ein weiterer Gegenstand der vorliegenden Erfindung ein Verfahren zur Herstellung des erfindungsgemäßen Formkörpers umfassend die folgenden Schritte:
a) Bereitstellen eines Schlickers umfassend ein keramisches Material;
b) Aufbringen des Schlickers auf ein Trägermaterial in einer gewünschten Schichtdicke;
c) Trocknen der Schicht aus Schritt b) unter Erhalt einer einzelnen keramischen Lage;
d) Wiederholen der Schritte a) bis c) bis die gewünschte Anzahl an keramischen Lagen erreicht ist;
e) Anordnen der einzelnen Lagen aus Schritt d) unter Erhalt einer Stapelanordnung; und
f) Pressen der Stapelanordnung aus Schritt e) unter Erhalt eines Formkörpers.

In einer bevorzugten Ausführungsform wird der Formkörper gesintert. Auf diese Weise lässt sich eine ausreichende Stabilität des keramischen Gefüges erreichen. In einer alternativ bevorzugten Ausführungsform wird die nicht gesinterte Stapelanordnungen entbindert oder ausgewaschen oder durch Lösung in geeigneten Lösungsmitteln von organischem Material befreit. Auch diese gradierten Strukturen mit Porositäts- oder Materialgradienten können im Anschluss infiltriert werden und weisen dann einen Gradienten an Füllkörpern im dichten infiltrierten Gefüge auf.

In einer bevorzugten Ausführungsform umfasst der Schlicker Keramikpulver, Dispergierhilfen und eine flüssige Phase. Dieser erste Schlicker kann beispielsweise durch Mahlung und Homogenisierung der Schlickerbestandteile hergestellt werden. Durch Zugabe weiterer Bestandteile wie beispielsweise rheologischen Hilfsmittel, Verflüssigungsmitteln, Entschäumern, Bindern und/oder Vernetzungsmitteln wird vorzugsweise durch Rühren ein zweiter Schlicker hergestellt, der auf das Trägermaterial aufgebracht wird.

In einer bevorzugten Ausführungsform handelt es sich bei der flüssigen Phase des Schlickers um Wasser, anorganische oder organische Lösungsmittel oder Mischungen hiervon.

In einer bevorzugten Ausführungsform erfolgt das Aufbringen des Schlickers auf das Trägermaterial mittels Rakeln, Sprühen oder Gießen. Ohne auf bestimmte Verfahren zum Aufbringen beschränkt zu sein, kommen beispielhaft etablierte Verfahren, wie etwa das Spin-coating, Dip-coating, Doktor Blade, Slot-coating oder auch Spray-coating in Frage. Auf diese Weise kann die gewünschte Schichtdicke optimal eingestellt werden.

Die getrocknete Schicht sollte sich zerstörungsfrei und rückstandsfrei von dem Trägermaterial ablösen lassen. Vorzugsweise ist das Trägermaterial ausgewählt aus der Gruppe bestehend aus Zellophanfolie, Kunststoffplatten oder-folien aus verschiedenen Materialien in verschiedenen Stärken, saugfähigem oder nicht saugfähigem, gegebenenfalls beschichtetem Papier, Metallfolien oder -blechen, Membranen zur Flüssigkeitsentfernung und ähnliche Trägermaterialien.

In einer bevorzugten Ausführungsform kann der erhaltene Formkörper nach dem Pressen bearbeitet werden, beispielsweise mittels subtraktiver Verfahren wie CAD/CAM-Verfahren oder Laser-Milling. Die Bearbeitung kann aber auch manuell, beispielsweise mittels handgeführten Fräswerkzeugen, erfolgen. In einer alternativ bevorzugten Ausführungsform kann der gepresste Formkörper mittels additiver Verfahren bearbeitet werden.

Je nach verwendetem Material kann es vorteilhaft sein, den Formkörper vor der Bearbeitung vorzusintern, um so die Bearbeitung zu erleichtern. Daher ist eine Ausführungsform des erfindungsgemäßen Verfahrens bevorzugt, bei der der Formkörper nach dem Pressen und vor der Verarbeitung vorgesintert wird, wobei die erreichte Dichte kleiner als die endgültige Dichte ist.

Die gewünschte Festigkeit des Formkörpers kann je nach eingesetztem keramischem Material durch Sintern erreicht werden. Entsprechend ist eine Ausführungsform bevorzugt, bei der der Formkörper einem Sinterschritt unterzogen wird. Die Sinterung kann beispielsweise nach der Bearbeitung des Formkörpers erfolgen. Alternativ kann die Sinterung vor der Bearbeitung des Formkörpers erfolgen.

Die zu erreichende Dichte hängt von der gewünschten Verwendung des Formkörpers ab. Beispielsweise kann der Formkörper auf mehr als 70% seiner theoretischen Dichte, beispielsweise mehr als 80% seiner theoretischen Dichte oder mehr als 90% seiner theoretischen Dichte gesintert werden. Hohe Dichten, die mit einer hohen Festigkeit in Zusammenhang gebracht werden, sind insbesondere bei der Verwendung des Formkörpers im Bereich der Dentalrestaurationen wünschenswert. Für andere Anwendungen kann es von Vorteil sein, den Formkörper auf beispielsweise 30% seiner theoretischen Dichte zu sintern.

Durch das Sintern des bearbeiteten Formkörpers kann es zu einer Schrumpfung desselben kommen, daher ist eine Ausführungsform bevorzugt, in der dieser Schrumpfungsfaktor bereits bei der Bearbeitung, beispielsweise dem Ausformen einer dentalen Restauration, berücksichtigt ist, beispielsweise indem die dentale Restauration um diesen Schrumpfungsfaktor vergrößert wird.

Zur besseren Verarbeitbarkeit kann dem Schlicker ein Bindemittel beigemischt werden. In einer bevorzugten Ausführungsform umfasst das erfindungsgemäße Verfahren daher einen Schritt des Entbinderns, in dem das Bindemittel, beispielsweise durch thermische Behandlung, nach dem Pressen des Formkörpers entfernt wird.

Das erfindungsgemäße Verfahren erlaubt die Herstellung eines porösen oder teilporösen Formkörpers, in dessen Poren ein oder mehrere weitere Materialien eingebracht werden können. Zu diesem Zweck umfasst das erfindungsgemäße Verfahren in einer bevorzugten Ausführungsform einen Schritt der Silanisierung. Es wurde überraschend gefunden, dass durch die Silanisierung der inneren Oberfläche des Formkörpers ein besserer chemischer Verbund mit dem weiteren Material erreicht wird, das in die Poren des Formkörpers eingebracht wird. Die Silanisierung erfolgt vorzugsweise durch in Kontakt bringen des Formkörpers mit einer Lösung umfassend eine siliziumhaltige Verbindung, vorzugsweise ein Silan. Die Lösung kann dabei wässrig, teilwässrig oder nichtwässrig sein. Vorzugsweise wird der Formkörper nach der Silanisierung einem Trocknungsschritt unterzogen

Das Einbringen des weiteren Materials in den Formkörper erfolgt vorzugsweise nach der Silanisierung und anschließenden Trocknung des Formkörpers. Vorzugsweise erfolgt das Einbringen durch in Kontakt bringen des Formkörpers mit dem weiteren oder den weiteren Materialien. Bei dem weiteren Material kann es sich beispielsweise um ein Monomer oder um eine Monomermischung handeln, wobei das Einbringen optional in Gegenwart eines Polymerisationsstarters erfolgt. Vorzugsweise kann dem Monomer oder der Monomermischung ein oder mehrere Füllstoffe beigemischt werden, beispielsweise Füllstoffe zur Beeinflussung der physikalischen Eigenschaften wie Farbe, Transluzenz, Biegefestigkeit, Röntgenopazität, Opaleffekt und/oder Fluoreszenz. Beispielsweise kann es sich bei dem Füllstoff um farbgebende Materialien wie organische, anorganische und/oder organisch-anorganische Mischpigmente handeln. Das eingebrachte Monomer und/oder die eingebrachte Monomermischung kann dann in einem anschließenden Schritt polymerisiert werden.

Durch das erfindungsgemäße Verfahren werden dem Formkörper Eigenschaften aufgeprägt, die mittels herkömmlicher Verfahren nicht zugänglich sind, beispielsweise ein homogener Farbverlauf ohne für das bloße Auge erkennbare Übergänge. Daher ist ein weiterer Gegenstand der vorliegenden Erfindung ein mehrschichtiger Formkörper, erhalten durch das erfindungsgemäße Verfahren. Wie bereits ausgeführt, ist der erfindungsgemäße Formkörper besonders für Anwendungen auf dem Gebiet der dentalen Restauration geeignet. Daher ist ein weiterer Gegenstand der vorliegenden Erfindung die Verwendung des erfindungsgemäßen Formkörpers zur Herstellung einer dentalen Restauration. Es wurde überraschend gefunden, dass durch die erfindungsgemäße Verwendung dentale Restauration zur Verfügung gestellt werden können, die den natürlichen Farbverlauf der sie umgebenden Zähne nachahmen, ohne dass Farbübergänge für das menschliche Auge sichtbar wären.

Vorzugsweise handelt es sich bei der dentalen Restauration um künstliche Zähne, Inlays, Onlays, Brücken und/oder Kronen. Weiterhin bevorzugt erfolgt die Herstellung der dentalen Restauration computergestützt, insbesondere mittels CAD/CAM-Verfahren um die erforderliche Präzision zu erreichen.

Ein weiterer Gegenstand der vorliegenden Erfindung ist entsprechend eine Dentalrestauration erhältlich aus dem erfindungsgemäßen Formkörper. Es wurde überraschend gefunden, dass sich die erfindungsgemäßen Dentalrestaurationen durch einen natürlichen Farbverlauf ohne sichtbare Farbübergänge auszeichnen.

Ein weiterer Gegenstand der vorliegenden Erfindung ist ein Verfahren zur Herstellung einer dentalen Restauration unter Verwendung eines erfindungsgemäßen Formkörpers, wobei die dentale Restauration mittels abtragender Verfahren aus dem Formkörper geformt wird.

Die vorliegende Erfindung wird anhand der folgenden Beispiele verdeutlicht, wobei diese nicht als Einschränkung des Erfindungsgedankens aufzufassen sind.

Die allgemeine Vorgehensweise umfasst die Mahlung des Ausgangsmaterials zur Herstellung eines agglomeratfreien Schlickers. Dieser Schlicker wird mit Zusätzen zu einem gießfähigen Schlicker für den Folienguss eingestellt. Nach dem Ausbringen der gewünschten Schichtstärke mittels einer Rakel auf Zellophanfolie und dem Trocknen der so erzeugten keramischen Folien werden diese zugeschnitten und mit der gewünschten Abfolge aufeinandergestapelt und verpresst. Der anschließende Sinterschritt erzeugt den keramischen Block mit zu erreichender lokal verschiedener Porosität oder Farbe.

### Beispiel 1:

Feldspatkeramikpulver mit Korngröße D50 = 4,5 µm (L = large), farblos (A1a) und Zahnfarbe 2M2 (A1b):
Herstellung des Ausgangsschlickers A1a, bzw. A1b: Mahlen von 100 g Keramikpulver mit 0,6 g anionisches Polyelektrolyt (Dispergierhilfsmittel) und 81,5 g destilliertem Wasser mit 200 g ZrO₂-Mahlkugeln für 12 h, dann Absieben der Mahlkugeln.

Herstellung des Schlickers zum Foliengießen: 86,6 g Ausganggschlicker A1a, bzw. A1b, 2,68 g Ethylenvinylacetatdispersion (temporärer Binder), 0,54 g modifizierte Stärke (Binder / Verdickungsmittel), 5 g Ethanol/2%MEK (MEK = Methylethylketon) und 0,1 g 1-Octanol werden mit 500 rpm 1h gerührt, dann 20 sec. bei 2500 rpm im Speedmixer vermischt.

### Beispiel 2:

Feldspatkeramikpulver mit Korngröße D50 = 2,6 µm (M = medium), Zahnfarbe 3M2:
Herstellung des Ausgangsschlickers A2: Mahlen von 100 g Keramikpulver mit 0,6 g anionisches Polyelektrolyt und 81,5 g destilliertem Wasser mit 200 g ZrO₂-Mahlkugeln für 12 h, dann Absieben der Mahlkugeln.

Herstellung des Schlickers zum Foliengießen: 86,6 g Ausganggschlicker A2, 2,68 g Ethylenvinylacetatdispersion, 0,54 g modifizierte Stärke, 5 g Ethanol/2%MEK (MEK = Methylethylketon) und 0,1 g 1-Octanol werden mit 500 rpm 1h gerührt, dann 20 sec. bei 2500 rpm im Speedmixer vermischt.

### Beispiel 3:

Feldspatkeramikpulver mit Korngröße D50 = 1,4 µm (S = small), Zahnfarbe 2M2:
Herstellung des Ausgangsschlickers A3: Mahlen von 100 g Keramikpulver mit 0,6 g anionisches Polyelektrolyt und 81,5 g destilliertem Wasser mit 200 g ZrO₂-Mahlkugeln für 12 h, dann Absieben der Mahlkugeln.

Herstellung des Schlickers zum Foliengießen: 86,6 g Ausganggschlicker A3, 2,64 g Ethylenvinylacetatdispersion, 0,53 g modifizierte Stärke, 5 g Ethanol/2%MEK (MEK = Methylethylketon), 0,1 g 1-Octanol werden mit 500 rpm 1h gerührt, dann 20 sec. bei 2500 rpm im Speedmixer vermischt.

### Beispiel 4:

Zirkoniumdioxidpulver ZrO₂ TZ-PX-245 ex Tosoh yellow (B1a), ZrO₂ TZ-PX-364 ex Tosoh white (B1b):
Herstellung des Ausgangsschlickers B1a, bzw. B1b: Mahlen von 110 g ZrO₂-Pulver mit 1,0 g anionisches Polyelektrolyt und 60 g destilliertem Wasser mit 200 g ZrO₂-Mahlkugeln für 4 h, dann Absieben der Mahlkugeln.

Herstellung des Schlickers zum Foliengießen: 114 g Ausganggschlicker B1a, bzw. B1b, 4,73 g Ethylenvinylacetatdispersion, 1,3 g modifizierte Stärke, 13,3 g Ethanol/2%MEK (MEK = Methylethylketon), 0,1 g 1-Octanol werden mit 500 rpm 1h gerührt, dann 20 sec. bei 2500 rpm im Speedmixer vermischt.

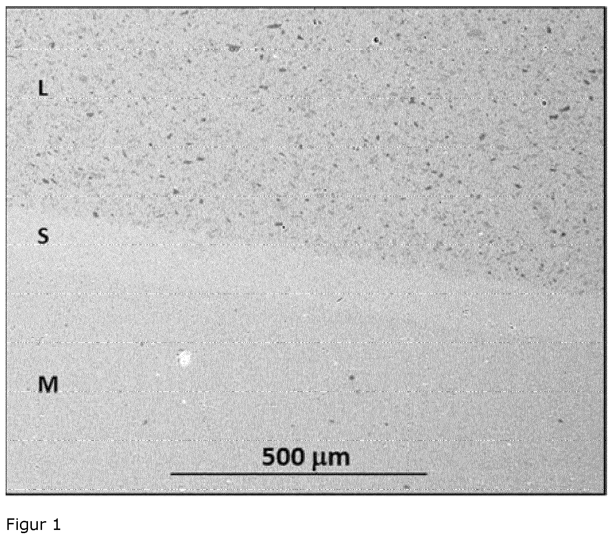

Figur 1 zeigt eine REM-Aufnahme eines erfindungsgemäßen Formkörpers, in dem klar der rissfreie Übergang von 4,5 µm- über 1,4 µm- zu 2,6 µm dicker Schichten in dicht gesintertem Zustand zu erkennen ist.

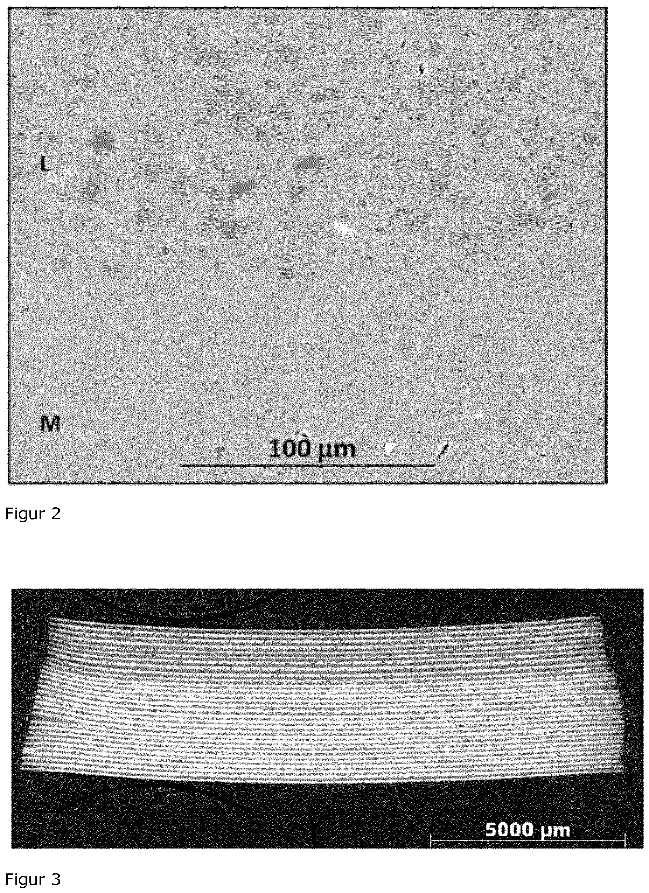

Figur 2 zeigt eine REM-Aufnahme eines weiteren erfindungsgemäßen Formkörpers, bei dem klar der rissfreie Übergang von 4,5 µm- zu 2,6 µm dicker Schichten in dicht gesintertem Zustand zu erkennen ist.
Figur 3 zeigt eine stereomikroskopische Aufnahme eines erfindungsgemäßen Formkörpers, der aus abwechselnden Lagen ZrO₂ und Feldspatkeramik besteht und gemäß dem erfindungsgemäßen Verfahren hergestellt wurde. Klar ist hier die Verbindung der beiden unterschiedlichen Materialien zu erkennen.

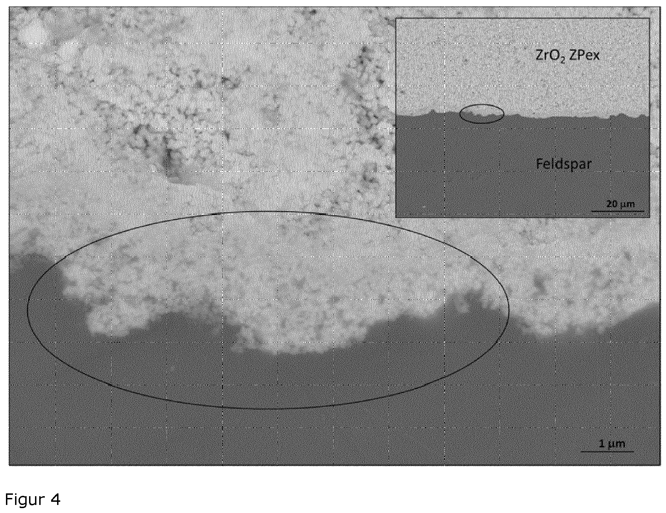

Figur 4 zeigt eine REM-BSD-Aufnahme eines erfindungsgemäßen Formkörpers, wobei die hervorgehobene Stelle den Übergang von porös gesintertem ZrO₂ zu Feldspatkeramik mit rissfreiem Verbund ohne Porosität zeigt.

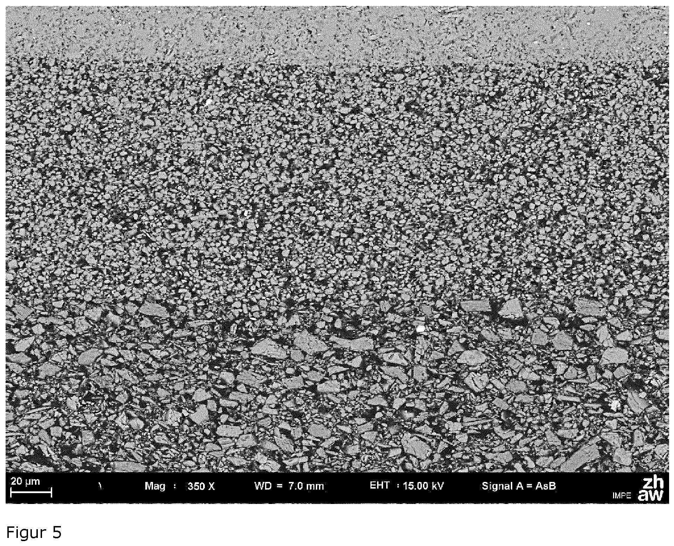

Figur 5 zeigt eine REM-Aufnahme eines erfindungsgemäßen Formkörpers aus porös gesinterter Feldspatkeramik in underschiedlichen Körnungen mit fast dichtem Gefüge (oben) und zwei unterschiedlichen Porositäten resultierend aus verschiedenen Korngrößen (Mitte und unten).

## Patentansprüche

1. Formkörper mit einer Vielzahl aufeinander angeordneter keramischer Lagen, wobei mindestens 21 Lagen aufeinander angeordnet sind.

2. Formkörper nach Anspruch 1, wobei die Lagen eine Stärke von 1 µm bis 120 µm, insbesondere 1 bis 99 µm, vorzugsweise 10 µm bis 80 µm, besonders bevorzugt 30 µm bis 60 µm aufweisen.

3. Formkörper nach Anspruch 1 oder 2, wobei die aufeinander angeordneten keramischen Lagen jeweils dasselbe keramische Material umfassen oder mindestens eine der aufeinander angeordneten keramischen Lagen ein von den anderen keramischen Lagen verschiedenes keramisches Material umfasst.

4. Formkörper nach mindestens einem der Ansprüche 1 bis 3, wobei das keramische Material ausgewählt aus der Gruppe bestehend aus Feldspatkeramik, Metalloxidkeramik, nicht-oxidische Keramik, Glaskeramik und Kombinationen davon ist.

5. Formkörper nach mindestens einem der Ansprüche 1 bis 4, wobei eine keramische Lage oder eine Mehrzahl von keramischen Lagen unterschiedliche Porositäten aufweisen.

6. Formkörper nach mindestens einem der Ansprüche 1 bis 5, wobei eine Mehrzahl von keramischen Lagen aus unterschiedlichen Materialien oder Materialien mit unterschiedlichen physikalischen Eigenschaften bestehen.

7. Formkörper nach mindestens einem der Ansprüche 1 bis 6, wobei mindestens eine der keramischen Lagen ein keramisches Material enthaltend mindestens einen Zuschlagstoff aufweist.

8. Formkörper nach Anspruch 7, wobei der Zuschlagstoff ausgewählt ist aus der Gruppe bestehend aus färbenden Substanzen, Keimbildnern, Flussmitteln, Läuterungsmitteln, keramischen Fasern, Nanomaterialien, Stabilisatoren und Mischungen hiervon.

9. Verfahren zur Herstellung eines Formkörpers nach mindestens einem der Ansprüche 1 bis 8, umfassend die Schritte:
a) Bereitstellen eines Schlickers umfassend ein keramisches Material;
b) Aufbringen des Schlickers auf ein Trägermaterial in einer gewünschten Schichtdicke;
c) Trocknen der Schicht aus Schritt b) unter Erhalt einer einzelnen keramischen Lage;
d) Wiederholen der Schritte a) bis c) bis die gewünschte Anzahl an keramischen Lagen erreicht ist;
e) Anordnen der einzelnen Lagen aus Schritt d) unter Erhalt einer Stapelanordnung; und
f) Pressen der Stapelanordnung aus Schritt e) unter Erhalt eines Formkörpers.

10. Verwendung eines Formkörpers nach mindestens einem der Ansprüche 1 bis 8 zur Herstellung einer dentalen Restauration.

11. Verwendung nach Anspruch 10, wobei die dentale Restauration künstliche Zähne, Inlays, Onlays, Brücken oder Kronen umfasst und/oder wobei die Herstellung der dentalen Restauration computergestützt durchgeführt wird.

12. Dentalrestauration erhältlich aus einem Formkörper nach mindestens einem der Ansprüche 1 bis 8.

13. Verfahren zur Herstellung einer Dentalrestauration unter Verwendung eines Formkörpers nach mindestens einem der Ansprüche 1 bis 8, wobei die Dentalrestauration mittels maschineller Bearbeitung aus dem Formkörper geformt wird.
